# EUROPÄISCHE PATENTSCHRIFT

(11) **EP 0 476 623 B1**
(45) Veröffentlichungstag und Bekanntmachung des Hinweises auf die Patenterteilung: **23.11.1994**
(21) Anmeldenummer: 91115856.6
(22) Anmeldetag: 18.09.1991
(51) Int. Cl.: A61F 5/37

(54) **Fixierbandage**
Fixation bandage
Bandage de fixation

(30) Priorität: 19.09.1990 DE 4029622
(43) Veröffentlichungstag der Anmeldung: 25.03.1992
(73) Patentinhaber: MIRO KLINIK-UND ÄRZTEBEDARF GmbH, D-51674 Wiehl (DE)
(72) Erfinder: Rothmann, Michael, W-5276 Wiehl 3 (DE)
(74) Vertreter: Patentanwälte Lippert, Stachow, Schmidt & Partner

(56) Entgegenhaltungen:
- EP-A- 0 198 482
- FR-A- 1 553 068
- US-A- 2 460 589
- US-A- 3 515 131

## Beschreibung

Die Erfindung betrifft eine Fixierbandage, insbesondere für Armfrakturen und Schulterluxationen, die als einteiliges, in Längsrichtung aus im wesentlichen undehnbarem Material gefertigtes Band ausgebildet ist.

Mit Hilfe eines solchen Fixierverbandes sollen der Arm, Oberarm und die Schulter sicher und dauerhaft ruhiggestellt werden. Bekannte Verbände erlauben zwar eine Fixierung von Oberarm und Schulter. Sie bringen jedoch auch erhebliche Nachteile für den Patienten und das Pflegepersonal mit sich.

Eine Fixierbandage der eingangs genannten Art ist aus der US-A-2,460,589 bekannt. Das einteilige Band führt an der die Verletzung aufweisenden Seite längs des Oberarms abwärts, bildet eine Schleife um die dem Ellenbogen benachbarte Partie des Unterarms und erstreckt sich auf der Rückseite des Patienten aufwärts zum Schulterteil des Bandes, an dem es befestigt ist. Von dieser Stelle aus verläuft das Schulterteil des Bandes diagonal über den Rücken des Patienten und von dort aus mit einer Schleife um den Handgelenkbereich des abgewinkelten verletzten Armes. Um eine seitliche Bewegung des Armes vom Körper weg zu vermeiden, ist ein zweites, separates Band vorgesehen, das oberhalb des Ellenbogens um den Oberarm geführt ist und dazu mit seinem einen Ende an der vorderseitigen Partie und mit seinem anderen Ende an der rückseitigen Partie des ersten Bandes befestigt ist.

Aufgrund der aneinander befestigten Bandbereiche bestimmter Längen ist die aus der US-A-2,460,589 bekannte Fixierbandage an eine bestimmte Oberarmlänge und -dicke angepaßt und nicht variierbar. Für verschiedene Größen müssen entsprechende Ausfertigungen der Fixierbandage bereitgestellt werden, um einen einwandfreien Sitz zu gewährleisten. Das vom Rücken her mit einer Schleife sich um das Handgelenk des verletzten Armes erstreckende Band kann sich durch Bewegung der Hand lockern und verrutschen, so daß der Unterarm nicht optimal ruhiggestellt ist.

Eine zufriedenstellende Festlegung des Armes ist mit einer Fixierbandage gemäß der EP 0 198 482 A1 möglich. Diese Fixierbandage besteht aus einem den Unter- und Oberarm aufnehmenden Schlauchteil, das schulterseits mit einem vorgefertigten Trageband und handseits mit einem vorgefertigten Halteband verbunden ist. Die beiden Bänder können auch zu einem einteiligen, durch das Schlauchteil geführten Band zusammengefaßt sein.

Diese bekannte Fixierbandage hat den Nachteil, daß aufgrund des den Unter- und Oberarm überziehenden Schlauchteils eine starke Schweißbildung unvermeidbar ist und heftiger Juckreiz und Allergien häufig auftreten. Zum Reinigen der Verletzung oder des Armes muß der Verband gelöst werden, was ebenfalls durch das Schlauchteil schwierig ist.

Außerdem führt das Trageband für den Unterarm bei einer Schulterverletzung genau über die verletzte Schulter und verursacht daher Druck und Schmerzen beim Patienten. Schließlich ist die Fertigbandage aufgrund der besonderen miteinander verbundenen Teile relativ teuer.

Frühere Fixierverbände waren einstückig aus einem Schlauchmaterial gefertigt, das geeignet zurecht geschnitten wurde. Dazu wurde ein Schlauchstück in ungefähr dreifacher Länge des verletzten Armes abgeschnitten. Das Schlauchstück wurde an seinen beiden Enden in Längsrichtung aufgeschnitten und danach über den Unter- und Oberarm gezogen. Das schulterseitige aufgeschnittene Ende wurde als Trageband über Nacken und Brust zum abgewinkelten Unterarm geführt und dort mit einer Schlaufe um den Unterarm oder das Handgelenk gelegt. Das handseitige aufgeschnittene Ende wurde etwa in Taillenhöhe über den Rücken geführt und mittels einer Schlaufe im unteren Bereich des Oberarms befestigt.

Diese Selbstanfertigung der früher verwendeten Fixierbandage war jedoch schwierig und zeitaufwendig. Weiterhin bestand die Gefahr, daß beim Zurechtschneiden des Verbandes nicht die erforderlichen Maße für das Schlauchstück und das Halte- und Trageband eingehalten wurden und der Verband daher nicht brauchbar war und neu angefertigt werden mußte.

Der vorliegenden Erfindung liegt die Aufgabe zugrunde, eine Fixierbandage der eingangs genannten Art zu schaffen, die sich durch eine verbesserte Wirtschaftlichkeit und Trageeigenschaft auszeichnet.

Die Aufgabe wird erfindungsgemäß dadurch gelöst, daß die Fixierbandage als einteiliges, in Längsrichtung aus im wesentlichen undehnbarem Material gefertigtes Band ausgebildet ist, das zur Verwendung als Bandage eine Länge aufweist, die es gestattet, das Band um den abgewinkelten Unterarm oder das Handgelenk zu legen und über die der verletzten Schulter, Arm oder Oberarm gegenüberliegende Seite zu führen, und von dort aus schräg über den Rücken und dann einmal in etwa Taillenhöhe um den Oberkörper herum bis zum Oberarm der betreffenden verletzten Körperseite und um diesen herum zu führen, und daß das Band an seinen beiden Enden Befestigungsmittel zur Befestigung der Enden am Band unter Bildung einer Schlaufe oder Manschette aufweist.

Da bei der erfindungsgemäßen Fixierbandage ein den Unter- und Oberarm aufnehmendes Schlauchteil vermieden wird, stellen sich auch die damit verbundenen Nachteile nicht mehr ein. Die aus einem einteiligen Band bestehende Bandage ist äußerst wirtschaftlich. Sie ist auf einfachste und den Patienten schonenste Weise anlegbar, und zwar auch dann, wenn sich der Patient unter Narkose befindet. Dieses einfache Anlegen der Fixierbandage geht in der Weise vor sich, daß das Band an seinem einen Ende unter Bildung einer Schlaufe um den abgewinkelten Unterarm oder das Handgelenk gelegt und das Ende mit Hilfe der Befestigungsmittel am Band befestigt wird, das Band über die der verletzten Schulter, dem verletzten Arm oder Oberarm gegenüberliegende Schulter, von dort aus schräg über den Rücken und dann einmal etwa in Taillenhöhe um den Oberkörper und über den den Unterarm bzw. das Handgelenk haltenden Teil des Bandes gelegt wird, das noch freie Ende des Bandes unter Bildung einer Manschette um den benachbarten Oberarm gelegt und mit Hilfe der Befestigungsmittel am Band befestigt wird.

Da der Unter- und Oberarm des Patienten bis auf die Manschette bzw. Schlaufe von Verbandmaterial freibleibt, werden eine verstärkte Schweißbildung und das Auftreten von Ekzemen, Allergien und dergleichen weitestgehend beseitigt.

Da die Verletzung frei zugänglich ist, kann diese ohne Abnehmen des Verbandes leicht kontrolliert und gepflegt werden. Zu Reinigungszwecken kann die Bandage durch Öffnen der um den Oberarm gelegten Manschette und der um den Unterarm oder das Handgelenk gelegten Schlaufe leicht und schonend gelöst werden. Da die Bandage über die der verletzten Schulter, dem verletzten Arm oder Oberarm gegenüberliegende Schulter geführt ist, werden Druck und Schmerzen an den verletzten Stellen beim Patienten vermieden. Die erfindungsgemäße Fixierbandage hat darüber hinaus auch den Vorteil, daß sie links und rechts tragbar ist.

Die angenehmen Trageeigenschaften der erfindungsgemäßen Fixierbandage können noch dadurch verbessert werden, daß das Band auf der dem Körper zuzuwendenden Innenseite ein Zellwollgewebe aufweist.

Vorzugsweise ist das Band dreischichtig aus einer Innenschicht aus Zellwollgewebe, einer weichen Polsterschicht als Zwischenlage und einer Außenschicht ausgebildet. Die Zwischenlage kann aus einer Schaumstoffschicht bestehen. In dieser Gestaltung weist das Band vorzugsweise eine Dicke von ca. 5 mm auf.

In einer weiteren zweckmäßigen Ausbildung ist das Band mit drei in Längsrichtung verlaufenden Steppnähten versehen, die ein Verrutschen der Fixierbandage verhindern. Die Befestigungsmittel an den Enden des Bandes weisen bevorzugt Klettverschlußelemente auf. Zur variablen Befestigung der Klettverschlußelemente auf der Außenschicht des Bandes bestehen diese vorzugsweise aus einem Flausch-Velours.

Vorzugsweise hat das Band eine Länge, die es gestattet, das Band an seinem einen Ende von innen nach außen unter Bildung einer Schlaufe um den abgewinkelten Unterarm oder das Handgelenk zu legen und das Ende mit Hilfe der Befestigungsmittel am Band zu befestigen, das Band über die der verletzten Schulter, dem verletzten Arm oder Oberarm gegenüberliegende Schulter und von dort aus schräg über den Rücken und dann einmal in etwa Taillenhöhe um den Oberkörper und über den den Unterarm bzw. das Handgelenk haltenden Teil des Bandes zu führen und das noch freie Ende des Bandes von innen nach außen unter Bildung einer Manschette um den benachbarten Oberarm zu legen und mit Hilfe der Befestigungsmittel am Band zu befestigen.

Ein bevorzugtes Ausführungsbeispiel der Erfindung wird im folgenden anhand der Zeichnung näher beschrieben. Es zeigen:
- Fig. 1: eine Fixierbandage im angelegten Zustand,
- Fig. 2: einen Querschnitt durch das Band und
- Fig. 3: eine perspektivische Ansicht eines Bandendes.

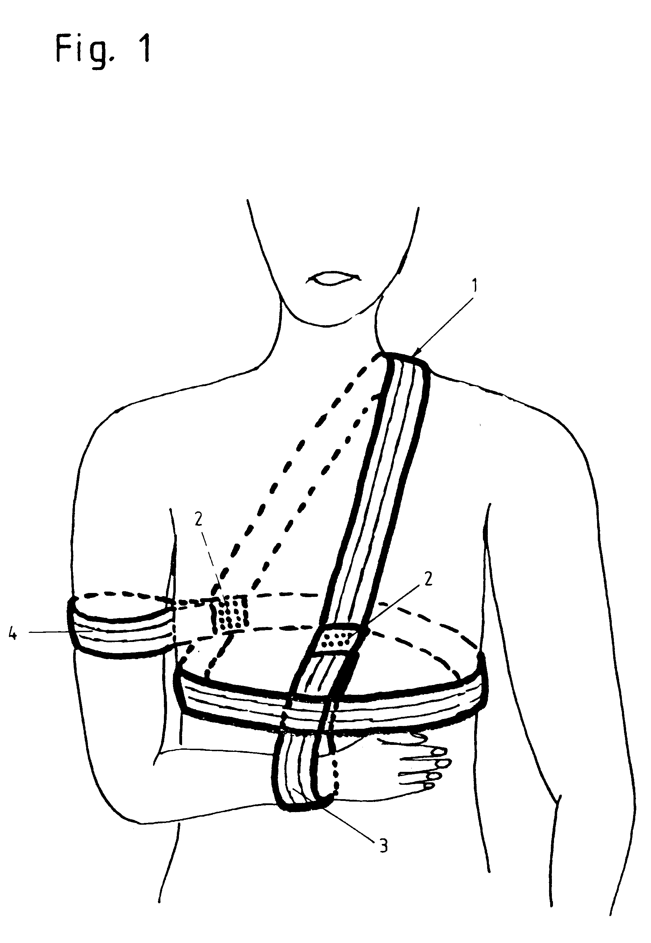

Wie in Fig. 1 dargestellt, besteht die Fixierbandage aus einem einteiligen Band 1, das an seinen beiden Enden geeignete Befestigungsmittel 2 zur Befestigung der Enden am Band 1 unter Bildung einer Schlaufe 3 um den Unterarm und einer Manschette 4 um den Oberarm des Patienten aufweist.

Das Band 1 ist über die der verletzten Schulter, dem verletzten Arm oder Oberarm gegenüberliegende Schulter gelegt und mit dem über die Brust herabführenden Ende von innen nach außen um den abgewinkelten Unterarm gelegt, wobei das Ende mit Hilfe der Befestigungsmittel 2 am Band 1 befestigt ist.

Von der Schulter führt das Band 1 andererseits schräg über den Rücken und ist dann einmal etwa in Taillenhöhe um den Oberkörper und über den den Unterarm haltenden Teil des Bandes gelegt.

Das andere Ende des Bandes ist unter Bildung der Manschette 4 von innen nach außen um den Oberarm geschlagen und mit Hilfe der Befestigungsmittel 2 am Band 1 befestigt.

Es ist ersichtlich, daß bei Verwendung einer derartigen Fixierbandage der Unter- und Oberarm des Patienten trotz einwandfreier Armfixierung freibleibt und somit eine stark störende Schweißbildung, Juckreiz und die Gefahr von Allergien vermieden werden. Weiterhin kann die Bandage zum Reinigen der Verletzung oder des Armes schnell und leicht und ohne Behinderung des Patienten gelöst werden. Aus Fig. 1 ist ferner ersichtlich, daß die verletzte Schulter bzw. der verletzte Arm oder Oberarm lediglich im Bereich der Manschette 4 vom Band 1 mit Druck beaufschlagt wird, so daß durch bisher bekannte Bandagen verursachte Schmerzen beim Patienten vermieden werden. Die Bandage eignet sich daher in optimaler Weise für alle postoperativen und posttraumatischen Verletzungen im Oberarm/Schulterbereich. Sie stellt somit eine Alternative zu Gilchrist- und Desaultverbänden dar.

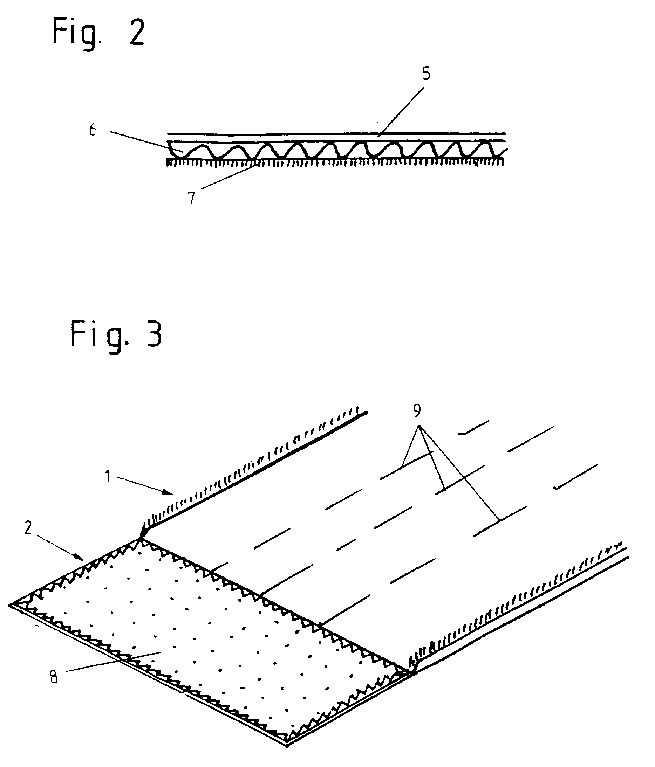

Wie aus Fig. 2 hervorgeht, hat das Band 1 einen dreischichtigen Aufbau mit einer hautfreundlichen Innenseite 5 aus Zellwollgewebe, einer weichen Polsterschicht 6 aus Schaumstoff und einer Außenschicht 7 aus einem Flausch-Velours. Aufgrund dieses Aufbaus hat das Band 1 eine Dicke von ca. 5 mm. Es zeichnet sich durch besonders angenehme Trageeigenschaften für den Patienten aus.

Wie aus Fig. 3 ersichtlich ist, hat das Band 1 an jedem seiner Enden ein sich über seine gesamte Breite erstreckendes Klettverschlußelement 8. Durch das Klettverschlußelement 8 kann das jeweilige Ende des Bandes 1 unter Bildung der Schlaufe 3 bzw. Manschette 4 variabel an der Außenschicht 7 aus Flausch-Velours befestigt werden.

Das auf diese Weise hergestellte Band 1 ist bei 30° waschbar und daher besonders wirtschaftlich.

Wie ebenfalls in Fig. 3 gezeigt ist, hat das Band 1 in Längsrichtung drei Steppnähte 9, die ein Verrutschen der Fixierbandage auf der Körperoberfläche des Patienten verhindern.

## Patentansprüche

1. Fixierbandage, insbesondere für Armfrakturen und Schulterluxationen, die als einteiliges, in Längsrichtung aus im wesentlichen undehnbarem Material gefertigtes Band ausgebildet ist, **dadurch gekennzeichnet**, daß das Band (1) zur Verwendung als Bandage eine Länge aufweist, die es gestattet, das Band (1) um den abgewinkelten Unterarm oder das Handgelenk zu legen und über die der verletzten Schulter, dem verletzten Arm oder Oberarm gegenüberliegende Schulter und von dort aus schräg über den Rücken und dann einmal in etwa Taillenhöhe um den Oberkörper herum bis zum Oberarm der betreffenden verletzten Körperseite und um diesen herum zu führen, und daß das Band (1) an seinen beiden Enden Befestigungsmittel (2) zur Befestigung der Enden am Band (1) unter Bildung einer Schlaufe (3) oder Manschette (4) aufweist.

2. Fixierbandage nach Anspruch 1, **dadurch gekennzeichnet**, daß das Band (1) auf der dem Körper zuzuwendenden Innenseite ein Zellwollgewebe aufweist.

3. Fixierbandage nach Anspruch 2, **dadurch gekennzeichnet**, daß das Band dreischichtig aus einer Innenschicht (5) aus Zellwollgewebe, einer weichen Polsterschicht (6) und einer Außenschicht (7) ausgebildet ist.

4. Fixierbandage nach einem der Ansprüche 1 bis 3, **dadurch gekennzeichnet**, daß das Band (1) in Längsrichtung mit einer oder mehreren Steppnähten (9) versehen ist.

5. Fixierbandage nach einem der Ansprüche 1 bis 4, **dadurch gekennzeichnet**, daß die Befestigungsmittel (2) an den Enden Klettverschlußelemente (8) aufweisen.

6. Fixierbandage nach Anspruch 5, **dadurch gekennzeichnet**, daß die Außenschicht (7) aus einem Flausch-Velours besteht.

## Claims

1. Immobilising bandage, in particular for fractures of the arm and dislocated shoulders, which is in the form of an integral strip produced in the longitudinal direction from material which is substantially non-expandable, characterised in that, in order to be used as a bandage, the length of the strip (1) enables the strip (1) to be laid about the bent lower arm or the wrist and guided over the shoulder opposite the injured shoulder, injured arm or upper arm and from there obliquely over the back and then once approximately at waist height about the upper body as far as the upper arm on the appropriate injured side of the body and about said upper arm; and in that the strip (1) comprises at both ends securing means (2) for securing the ends to the strip (1), forming a loop (3) or sleeve (4).

2. Immobilising bandage according to Claim 1, characterised in that the strip (1) comprises a staple fibre fabric on the interior side facing the body.

3. Immobilising bandage according to Claim 2, characterised in that the strip is formed in three layers of an inner layer (5) of staple fibre fabric, a further, padding layer (6) and an outer layer (7).

4. Immobilising bandage according to any one of Claims 1 to 3, characterised in that the strip (1) is provided in the longitudinal direction with one or a plurality of quilting seams (9).

5. Immobilising bandage according to any one of Claims 1 to 4, characterised in that the securing means (2) comprise at the ends loop and burr-type closure elements (8).

6. Immobilising bandage according to Claim 5, characterised in that the outer layer (7) consists of a fleecy-velour.

## Revendications

1. Bandage de contention, en particulier pour des fractures du bras et des luxations de l'épaule, réalisé sous forme de bande en une seule pièce, fabriquée en une matière pratiquement inextensible en direction longitudinale, caractérisé en ce que la bande (1) présente, pour son utilisation comme bandage, une longueur qui permet de la poser autour de l'avant-bras fléchi ou du poignet et de la faire passer sur l'épaule opposée à l'épaule blessée, au bras ou au segment supérieur du bras blessé et, à partir de la, obliquement sur le dos, puis une fois autour du buste à peu prés à la hauteur de la taille, jusqu'au segment supérieur du bras du côté blessé concerné du corps et autour de ce segment, et en ce que la bande (1) présente, à ses deux extrémités, des moyens de fixation (2) pour fixer ces extrémités a la bande (1) en formant une boucle (3) ou une manchette (4).

2. Bandage de contention selon la revendication 1, caractérisé en ce que la bande (1) comporte un tissu de laine cellulosique sur sa face interne, destinée à être appliquée contre le corps.

3. Bandage de contention selon la revendication 2, caractérisé en ce que la bande est formée de trois couches, à savoir une couche interne (5) en tissu de laine cellulosique, une couche molle de matelassage (6) et une couche externe (7).

4. Bandage de contention selon l'une quelconque des revendications 1 à 3, caractérisé en ce que la bande (1) est munie, en direction longitudinale, d'une ou de plusieurs coutures piquées (9).

5. Bandage de contention selon l'une quelconque des revendications 1 à 4, caractérisé en ce que les moyens de fixation (2) comportent, aux extrémités, des éléments de fermeture à ruban à boucles et crochets (8).

6. Bandage de contention selon la revendication 5, caractérisé en ce que la couche externe (7) est faite d'un velours à longs poils.
